# NOUVEAU FASCICULE DE BREVET EUROPEEN

(11) **EP 3 461 902 B2**
(45) Date de publication et mention de la décision concernant l'opposition: **03.01.2024**
(45) Mention de la délivrance du brevet: 02.12.2020
(21) Numéro de dépôt: 18191590.1
(22) Date de dépôt: 30.08.2018
(51) Int. Cl.: C12P 19/02, C12P 19/14, C12P 7/10

(54) **PROCÉDÉ D'HYDROLYSE ENZYMATIQUE EN ALIMENTATION SÉQUENTIELLE AVEC AJOUTS DU SUBSTRAT PRÉTRAITÉ DE PLUS EN PLUS ÉSPACES DANS LE TEMPS**
ENZYMATISCHES HYDROLYSEVERFAHREN MIT SEQUENTIELLER ZUFUHR VON ZUSÄTZEN VON VORBEHANDELTEM SUBSTRAT IN IMMER GRÖSSEREN ZEITABSTÄNDEN
PROCESS FOR ENZYMATIC HYDROLYSIS IN SEQUENTIAL FEEDING WITH ADDITION OF THE PRETREATED SUBSTRATE GRADUALLY SPACED OUT OVER TIME

(30) Priorité: 28.09.2017 FR 1759033
(43) Date de publication de la demande: 03.04.2019
(73) Titulaire: IFP Energies nouvelles, 92852 Rueil-Malmaison Cedex (FR)
(72) Inventeur: BATTISTA, Federico, 73026 Melendugno (Lecce) (IT); GOMEZ ALMENDROS, Mélanie, 69003 LYON (FR); ROUSSET, Romain, 69600 OULLINS (FR)
(74) Mandataire: IFP Energies nouvelles

(56) Documents cités:
- WO-A1-2015/107415
- CN-A- 104 651 429
- ROSGAARD L ET AL: "Effects of substrate loading on enzymatic hydrolysis and viscosity of pretreated barley straw", APPLIED BIOCHEMISTRY AND BIOTECHNOLOGY ; PART A: ENZYME ENGINEERING AND BIOTECHNOLOGY, HUMANA PRESS INC, NEW YORK, vol. 143, no. 1, 1 octobre 2007 (2007-10-01), pages 27-40, XP002668850, ISSN: 0273-2289, DOI: 10.1007/S12010-007-0028-1 [extrait le 2007-04-17]
- OLOFSSON ET AL: "Designing simultaneous saccharification and fermentation for improved xylose conversion by a recombinant strain of Saccharomyces cerevisiae", JOURNAL OF BIOTECHNOLOGY, ELSEVIER, AMSTERDAM, NL, vol. 134, no. 1-2, 17 janvier 2008 (2008-01-17), pages 112-120, XP022500133, ISSN: 0168-1656
- MODENBACH ALICIA A ET AL: "Enzymatic hydrolysis of biomass at high-solids loadings - A review", BIOMASS AND BIOENERGY, vol. 56, 2013, pages 526-544, XP028684730, ISSN: 0961-9534, DOI: 10.1016/J.BIOMBIOE.2013.05.031
- SOTANIEMI VILLE-HERMANNI ET AL: "Controlled feeding of lignocellulosic substrate enhances the performance of fed-batch enzymatic hydrolysis in a stirred tank reactor", BIOMASS AND BIOENERGY, PERGAMON, AMSTERDAM, NL, vol. 91, 6 juin 2016 (2016-06-06), pages 271-277, XP029619781, ISSN: 0961-9534, DOI: 10.1016/J.BIOMBIOE.2016.05.037
- ZHANG X ET AL: "High consistency enzymatic hydrolysis of hardwood substrates", BIORESOURCE TECHNOLOGY, ELSEVIER, AMSTERDAM, NL, vol. 100, no. 23, 1 décembre 2009 (2009-12-01), pages 5890-5897, XP026469499, ISSN: 0960-8524, DOI: 10.1016/J.BIORTECH.2009.06.082 [extrait le 2009-07-29]
- FEDERICO BATTISTA ET AL: "Enzymatic hydrolysis at high dry matter content: The influence of the substrates' physical properties and of loading strategies on mixing and energetic consumption", BIORESOURCE TECHNOLOGY, vol. 250, 20 novembre 2017 (2017-11-20), pages 191-196, XP055480461, AMSTERDAM, NL ISSN: 0960-8524, DOI: 10.1016/j.biortech.2017.11.049
- Corrêa Luciano Jacob; Badino Alberto Colli; Cruz Antonio José: "Power consumption evaluation of different fed-batch strategies for enzymatic hydrolysis of sugarcane bagasse", BIOPROCESS AND BIOSYSTEMS ENGINEERING, SPRINGER, DE, vol. 39, no. 5, 22 February 2016 (2016-02-22), pages 825-833, DE ISSN: 1615-7591, DOI: 10.1007/s00449-016-1562-4
- Corrêa Luciano Jacob; Badino Alberto Colli; Cruz Antonio José: "Mixing design for enzymatic hydrolysis of sugarcane bagasse: methodology for selection of impeller configuration", BIOPROCESS AND BIOSYSTEMS ENGINEERING, SPRINGER, DE, vol. 39, no. 2, 9 December 2015 (2015-12-09), pages 285-294, DE ISSN: 1615-7591, DOI: 10.1007/s00449-015-1512-6
- Liu Yunyun; Xu Jingliang; Zhang Yu; Yuan Zhenhong; Xie Jun: "Optimization of high solids fed-batch saccharification of sugarcane bagasse based on system viscosity changes", Journal of Biotechnology, Elsevier, Amsterdam NL, vol. 211, 3 July 2015 (2015-07-03), pages 5-9, Amsterdam NL ISSN: 0168-1656, DOI: 10.1016/j.jbiotec.2015.06.422
- CUI et al.: "Enhanced enzymatic hydrolysis of lignocellulose by integrated decrystallization and fed-batch operation", RSC Adv., vol. 4, no. 84, 11 September 2014 (2014-09-11), pages 44659-44665,
- Zhao Xuebing; Dong Lei; Chen Liang; Liu Dehua: "Batch and multi-step fed-batch enzymatic saccharification of Formiline-pretreated sugarcane bagasse at high solid loadings for high sugar and ethanol titers", Bioresource Technology, ELSEVIER, AMSTERDAM, NL, vol. 135, 29 September 2012 (2012-09-29), pages 350-356, AMSTERDAM, NL ISSN: 0960-8524, DOI: 10.1016/j.biortech.2012.09.074
- Yu Zhang, Liu Yun-Yun, Xu Jing-Liang, Yuan Zhen-Hong, Qi Wei, Zhuang Xin-Shu, He Min-Chao: "High solid and low enzyme loading based saccharification of agricultural biomass", BioResources, vol. 7, no. 1, 1 January 2012 (2012-01-01) , pages 345-353,
- YANG et al.: "High-concentration sugars production from corn stover based on combined pretreatments and fed-batch process", Bioresour. Tech., vol. 101, no. 13, 12 January 2010 (2010-01-12), pages 4884-4888,
- David B. Hodge, M. Nazmul Karim, Daniel J. Schell, James D. Mcmillan: "Model-Based Fed-Batch for High-Solids Enzymatic Cellulose Hydrolysis", Applied Biochemistry and Biotechnology, Humana Press, vol. 152, no. 1, 1 January 2009 (2009-01-01), pages 88-107, ISSN: 02732289, DOI: 10.1007/s12010-008-8217-0
- M.J. Cardona, E.J. Tozzi, N. Karuna, T. Jeoh, R.L. Powell, M.J. Mccarthy: "A process for energy-efficient high-solids fed-batch enzymatic liquefaction of cellulosic biomass", Bioresource Technology, ELSEVIER, AMSTERDAM, NL, vol. 198, 1 December 2015 (2015-12-01), pages 488-496, AMSTERDAM, NL ISSN: 0960-8524, DOI: 10.1016/j.biortech.2015.09.042
- Tozzi et al., Aiche Journal, 2014, 60/5, 1582-15900
- Jung et al., Appl. Biochem. Biotech., 2017,182,1108-20
- Modenbach et al.. Biomass & Bioen., 2013, 56, 526-544

## Description

### DOMAINE DE L'INVENTION

La présente invention concerne un procédé d'hydrolyse enzymatique à partir d'un substrat lignocellulosique prétraité en mode alimentation séquentielle, définie selon les revendications, permettant la transformation de la cellulose en glucose. Le glucose peut ensuite être utilisé dans diverses étapes ultérieures telles que par exemple dans une étape de fermentation pour la production d'alcools, ou pour la production d'intermédiaires pour la chimie.

### ART ANTÉRIEUR

La mise au point de procédés économiquement viables de valorisation de la biomasse lignocellulosique est aujourd'hui un vaste sujet d'actualité. La raréfaction des ressources fossiles et la concurrence avec les ressources alimentaires conduisent à chercher de nouvelles voies pour la production de biocarburants et d'intermédiaires chimiques.

Depuis les années 1970, la transformation de la biomasse ligno-cellulosique après hydrolyse des polysaccharides constitutifs en sucres a fait l'objet de nombreux travaux.

La biomasse lignocellulosique se caractérise par une structure complexe constituée de trois principaux polymères : la cellulose, les hémicelluloses et la lignine, dont la proportion varie suivant les espèces de biomasse lignocellulosique. Une composition typique mais non limitative est la suivante : la cellulose est présente à hauteur de 35 à 50%, les hémicelluloses qui sont des polysaccharides essentiellement constitués de pentoses et d'hexoses sont présents à hauteur de 20 à 30% et les lignines à hauteur de 15 à 25% poids. La dégradation de la biomasse se révèle difficile car les polysaccharides de la paroi végétale (cellulose et hémicelluloses) sont intimement associés à de la lignine qui confère aux parois leur rigidité.

De ces trois polymères, la cellulose est la principale source de sucres car elle est constituée de glucose, ce dernier pouvant être facilement valorisé.

De façon classique, les procédés de valorisation de la biomasse par voie biochimique comprennent plusieurs étapes. Une première étape est la collecte et le transport de la biomasse lignocellulosique dans un centre de transformation de la biomasse. La deuxième étape est le prétraitement ou préhydrolyse de la biomasse qui permet de rendre la cellulose accessible aux enzymes et ainsi de produire un substrat lignocellulosique prétraité. La troisième étape d'hydrolyse enzymatique permet, grâce à l'utilisation d'une solution d'enzymes cellulolytiques et hémicellulolytiques produites par des microorganismes, et appelée cocktail enzymatique, la transformation de la cellulose en glucose. Ce glucose peut être ensuite valorisé en produits intermédiaires par exemple en éthanol lors d'une quatrième étape de fermentation par, en général, la levure *Saccharomyces cerevisiae,* ou en mélange acétone, butanol, éthanol (ABE) par fermentation par la levure *Clostridium acetobutylicum.* Une cinquième étape de distillation permet ensuite de concentrer les molécules obtenues. Le glucose peut également être valorisé en biocarburants (hydrogène, méthane).

Une des étapes clés est ainsi l'hydrolyse enzymatique. Dans l'étape d'hydrolyse enzymatique, ledit substrat lignocellulosique prétraité doit être mélangé avec une solution liquide contenant les enzymes cellulolytiques et hémicellulolytiques. L'objectif étant d'obtenir une concentration élevée en sucres, l'étape d'hydrolyse enzymatique doit se faire à des concentrations élevées en substrat lignocellulosique prétraité, c'est-à-dire à haute teneur en matière sèche. Il a été évalué que le procédé est économiquement viable, lorsque une concentration minimale de sucres de 8% poids est produite lors de l'hydrolyse enzymatique, ce qui ramène à une teneur en matière sèche d'environ 15 % poids (Mclntosh, S., Zhang, Z., Palmer, J., Wong, H., Doherty, W.O.S., Vancov, T., 2016. Pilot-scale cellulosic ethanol production using eucalyptus biomass pre-treated by dilute acid and steam explosion. Biofules, bioproducts and biorefining 10 (4), 346-358). Le travail à une teneur élevée de matière sèche permet également de réduire le volume du réacteur et, par conséquent, de réduire les coûts économiques et énergétiques du processus (Larsen, J., Ostergaard Petersen, M., Thirup, L., Wen Li, H., Krogh Iversen, F., 2008.

The IBUS process of lignocellulosic bioethanol close to a commercial reality.Chem. Eng. Technol. 31, 765-722).

Cependant, le mélange intime du substrat lignocellulosique prétraité avec ladite solution liquide contenant les enzymes cellulolytiques et hémicellulolytiques s'avère difficile lorsque les teneurs en matière sèche sont élevées. En effet, le début de l'hydrolyse enzymatique à haute teneur en matière sèche notamment pose des problèmes de mélange et d'homogénéisation. Le milieu réactionnel est très pâteux et visqueux ce qui demande une agitation spécifique beaucoup plus complexe que celle nécessaire en fin d'hydrolyse où le mélange réactionnel est devenu plus liquide.

D'une manière générale, l'hydrolyse enzymatique peut être effectuée dans des réacteurs discontinus ou continus. Dans un procédé discontinu, ou batch selon la terminologie anglo-saxonne, tous les composants, y compris les substances qui contrôlent le pH, sont mis dans le réacteur au début de l'hydrolyse. Pendant le procédé d'hydrolyse, il n'y a pas d'entrée ou de sortie du réacteur. Dans un procédé continu, il y a à la fois des flux d'entrée et de sortie, mais le volume de réaction est maintenu constant.

Dans une autre configuration de procédé, aussi appelé procédé avec alimentation séquentielle ou procédé en mode « fed-batch » selon la terminologie anglo-saxonne, rien n'est éliminé du réacteur pendant le procédé, mais le substrat est progressivement ajouté de manière séquentielle dans le réacteur pendant la période d'hydrolyse sans retirer d'hydrolysat. On a constaté que ce type d'alimentation des substrats permet de surmonter des effets tels que l'inhibition du substrat sur le rendement du produit. Au fur et à mesure de l'avancée de la réaction, le mélange devient de plus en plus liquide et il est possible d'ajouter du substrat frais de manière à monter le taux de matière sèche. Il est alors possible d'atteindre des concentrations en substrat élevées et avantageusement compris entre 17 et 30 % poids de matière sèche.

Des procédés d'hydrolyse enzymatique avec alimentation continue sont connus dans l'état de l'art (Mondebach, A.A., Nokel, S.E., 2013. Enzymatic hydrolysis of biomass at high-solids loadings - A Review. Biomass and Bioenergy 56, 526-544).

De même, la demande de brevet US2010/330638A décrit une alimentation en mode « fed-batch » de l'étape d'hydrolyse enzymatique, en indiquant que des tests permettent de déterminer la quantité de biomasse qui peut être ajoutée à chaque batch. Il est donc nécessaire d'effectuer des tests préalables à l'étape d'hydrolyse enzymatique lors de chaque changement de type de substrat.

La demande WO2016/062646 décrit un procédé de préparation d'un sucre et/ou d'un produit de fermentation à partir de matière lignocellulosique qui comprend plusieurs étapes d'hydrolyse enzymatique dont la première en mode « fed-batch ».

La demande de brevet US 2010/0255554 décrit un procédé d'hydrolyse de la biomasse lignocellulosique en mode « fed-batch » dans lequel les paramètres de fonctionnement du procédé sont ajustés en contrôlant le volume du réacteur et/ou la fréquence d'addition de la charge biomasse lignocellulosique prétraitée et éventuellement l'ajout d'enzymes, et le volume et/ou la concentration en sucres produits dans le réacteur. En particulier, la charge biomasse lignocellulosique prétraitée est toujours ajoutée à la même fréquence dans le réacteur.

La demanderesse a mis au point un procédé d'hydrolyse enzymatique avec alimentation séquentielle (« fed-batch ») amélioré permettant d'obtenir des rendements élevés en glucose tout en réduisant la consommation énergétique du procédé et le temps de mélange.

Plus particulièrement, la présente invention porte sur un procédé d'hydrolyse enzymatique en alimentation séquentielle dans lequel on met en contact, sous agitation, au moins un substrat lignocellulosique prétraité avec de l'eau et avec des enzymes dans un réacteur, ledit procédé étant caractérisé en ce que l'ajout séquentiel dans le réacteur du substrat lignocellulosique prétraité est effectué de façon de plus en plus espacé dans le temps, de manière à obtenir un taux de matière sèche final prédéterminé compris entre 18 et 30% poids, dans lequel :
le taux de matière sèche en début du procédé est inférieur à 10 % poids,
le réacteur comprend un agitateur, la vitesse de rotation dudit agitateur étant inférieure à 100 rpm et le rapport diamètre de l'agitateur/diamètre du réacteur D/T étant compris entre 0,3 et 0,75.

Lorsque le substrat lignocellulosique prétraité, et de préférence également les enzymes, sont injectés dans un procédé d'hydrolyse enzymatique avec alimentation séquentielle (« fed-batch ») de façon de plus en plus espacée dans le temps, on observe une augmentation du rendement en glucose ainsi qu'une baisse en consommation d'énergie par rapport à un procédé « fed-batch » dans lequel le substrat est ajouté d'une façon régulière dans le temps.

De plus, lorsque les enzymes sont ajoutées de la même manière, c'est-à-dire séquentielle et espacée dans le temps, et de préférence en même temps que le substrat, on observe un effet synergique dans le rendement en glucose qu'on n'observe pas lorsque la totalité des enzymes est ajoutée en début de l'hydrolyse enzymatique.

Le document de Rosgaard L. et al. 2007: "Effects of substrate loading on enzymatic hydrolysis and viscosity of pretreated barley straw", in Applied Biochemistry and Biotechnology, part A, 143(1): 27-40), décrit un procédé d'hydrolyse enzymatique de biomasse lignocellulosique dont la biomasse est ajoutée soit en une fois au départ, soit en deux fois, soit en trois fois, avec des espacements entre deux ajouts croissantes sur un période de 72 heures. Cependant, les résultats indiquent que l'efficacité de l'opération d'hydrolyse enzymatique est moins élevée avec des ajouts séquentiels.

Un avantage de la présente invention est de fournir un procédé d'hydrolyse enzymatique dans lequel le rendement de glucose est amélioré.

Un autre avantage de la présente invention est de fournir un procédé d'hydrolyse enzymatique dans lequel, grâce à la technique « fed-batch » mais de façon espacée dans le temps, les problèmes de mélange et de viscosité ne sont pas ou moins observés. En effet, grâce à l'augmentation du taux de matière sèche progressive, le mélange se fait facilement à chaque ajout de substrat, ce qui permet d'un côté de réduire la consommation énergétique des agitateurs et/ou d'utiliser des agitateurs plus simple, de type agitateurs à pâles inclinées (aussi connu sous le nom « Inclined Blades Impeller » selon la terminologie anglo-saxonne) ou encore de type hélice marine (aussi connu sous le nom « Marine Impeller » selon la terminologie anglo-saxonne).

De plus, un autre avantage de la présente invention est de fournir un procédé d'hydrolyse enzymatique dans lequel la vitesse de rotation de l'agitateur est faible, ce qui est important pour maintenir l'activité enzymatique (Mhlongo Sl, Haan R, Viljoen-Bloom M, Zyl WH. Lignocellulosic hydrolysate inhibitors selectively inhibit/deactivatecellulase performance (2015). Enzyme and Microbial Technology, 81: 16-22).

De plus, le procédé selon l'invention est adapté dans le cas où plusieurs substrats de nature différente sont traités simultanément dans le même réacteur (co-processing).

Un autre avantage de la présente invention est de fournir un procédé d'hydrolyse enzymatique permettant un suivi et une adaptation simple à l'évolution du milieu réactionnel ne nécessitant pas de mesures complexes.

Selon une variante, le taux de matière sèche final est compris entre 18 et 24% poids.

Selon une variante, l'ajout des enzymes dans le réacteur est effectué d'une façon séquentielle et de plus en plus espacé dans le temps.

Selon une variante, le substrat lignocellulosique prétraité et les enzymes sont ajoutés en même temps dans le réacteur.

Selon une variante, à chaque ajout, le substrat lignocellulosique prétraité est ajouté en quantité égale.

Selon une variante, à chaque ajout, les enzymes sont ajoutées en quantité égale.

Le réacteur comprend un agitateur et le rapport diamètre de l'agitateur/diamètre du réacteur D/T est compris entre 0,3 et 0,75.

Selon une variante, l'agitateur est un agitateur à pâles inclinées ou encore de type hélice marine.

Selon une variante, les enzymes sont mises en contact à une concentration comprise entre 0,1 et 60 mg d'enzymes par gramme de cellulose.

Selon une variante, le procédé opère à une température comprise entre 40 et 60°C, à un pH compris entre 4 et 6, et à pression atmosphérique.

Selon une variante, différents substrats lignocellulosiques prétraités sont utilisés, seuls ou en mélange.

Selon une variante, ledit procédé est suivi d'une étape de fermentation en présence d'un microorganisme alcooligène.

Selon une autre variante, ledit procédé est réalisé en présence d'un microorganisme alcooligène selon un procédé de saccharification et de fermentation simultanées dit procédé SSF.

### DESCRIPTION DÉTAILLÉE DE L'INVENTION

La biomasse lignocellulosique prétraitée est obtenue à partir de bois (feuillus et résineux), brut ou traité, de sous-produits de l'agriculture tels que la paille, de fibres de plantes, de cultures forestières, de résidus de plantes alcooligènes, sucrières et céréalières, de résidus de l'industrie papetière, de biomasse marine (par exemples macroalgues cellulosiques) ou de produits de transformations de matériaux lignocellulosiques.

Préférentiellement, la biomasse lignocellulosique utilisée est du bois, de la paille de blé, de la pulpe de bois, du miscanthus, de la paille de riz, ou des tiges de maïs.

Selon le procédé de l'invention, les différents types de biomasse lignocellulosique peuvent être utilisés seuls ou en mélange.

Les substrats lignocellulosiques utilisés dans le procédé de l'invention sont issus de la biomasse prétraitée dans des conditions permettant de déstructurer la lignocellulose en modifiant les propriétés physiques et physico-chimiques du matériau lignocellulosique. L'étape de prétraitement peut se faire selon tous types de prétraitement de biomasse lignocellulosique connus de l'homme du métier. Une étape de conditionnement au préalable, incluant par exemple un broyage ou un épierrage peut être aussi réalisée. L'étape de prétraitement peut être un traitement thermique, chimique, mécanique et/ou enzymatique ou une combinaison de ces traitements.

Selon une variante préférée, l'étape de prétraitement est choisie parmi un prétraitement en conditions acides tels qu'une cuisson acide ou l'explosion à la vapeur en conditions acides, un prétraitement en milieux alcalins tels qu'un prétraitement au sulfure de sodium (procédé Kraft), un procédé ARP (selon la terminologie anglo-saxonne Ammonia Recycle Percolation) ou un procédé AFEX (selon la terminologie anglo-saxonne Ammonia Fiber Explosion), un prétraitement oxydant tels qu'un prétraitement utilisant l'ozone, le peroxyde d'hydrogène, l'oxygène ou l'acide peracétique, un prétraitement sans ajout de réactifs chimiques tel que l'explosion à la vapeur sans ajout d'acide ou le prétraitement par lavage à l'eau très chaude, ou encore un procédé organosolv.

Avantageusement, l'étape de prétraitement est un prétraitement par l'explosion à la vapeur en conditions acides. Dans les conditions optimales, de 150 à 250 °C pendant quelques minutes.

La présente invention porte sur un procédé d'hydrolyse enzymatique en alimentation séquentielle dans lequel on met en contact, sous agitation, un substrat lignocellulosique prétraité avec de l'eau et avec des enzymes dans un réacteur, ledit procédé étant caractérisé en ce que l'ajout séquentiel dans le réacteur du substrat lignocellulosique prétraité est effectué de façon de plus en plus espacé dans le temps, de manière à obtenir un taux de matière sèche final prédéterminé compris entre 18 et 30% poids, dans lequel :
le taux de matière sèche en début du procédé est inférieur à 10 % poids,
le réacteur comprend un agitateur, la vitesse de rotation dudit agitateur étant inférieure à 100 rpm et le rapport diamètre de l'agitateur/diamètre du réacteur D/T étant compris entre 0,3 et 0,75.

Le taux de matière sèche final prédéterminé est de manière préférée compris entre 18 et 24% poids. Dans toute la suite du texte, on exprime la concentration en substrat lignocellulosique prétraité en pourcentage poids de matière sèche. Le taux de matière sèche est mesuré selon la norme ASTM E1756-08(2015) « Standard Test Method for Determinatoin of Total Solids in Biomass ».

Le taux de matière sèche en début du procédé d'hydrolyse, lors du premier ajout du substrat lignocellulosique prétraité, est inférieur à 10 % poids, de préférence inférieur à 8% poids et de manière particulièrement préférée inférieur à 6% poids.

La fréquence de l'ajout du substrat lignocellulosique prétraité est de plus en plus espacée. On attend ainsi par des ajouts « de plus en plus espacés dans le temps» des ajouts qui se font avec une fréquence décroissante ou, autrement dit, avec une périodicité croissante. Ainsi, le temps écoulé entre le n-ième et le n+1 ième ajout est inférieur au temps écoulé entre le n+1 ième ajout et le n+2 ième ajout, et ainsi de suite. Par exemple, le temps écoulé entre le premier et le deuxième ajout de substrat est inférieur au temps écoulé entre le deuxième et le troisième ajout, et ainsi de suite. A titre d'exemple, le premier ajout peut se faire après 1 heure, le deuxième après 3 heures, le troisième après 6 heures, le quatrième après 13 heures et le cinquième après 24 heures.

Afin d'atteindre le taux de matière sèche final prédéterminé, on procède généralement, et de façon de plus en plus espacée, à au moins 3 ajouts de substrat, de préférence à au moins 4 ajouts de substrat, et de manière encore plus préférée à au moins 5 ajouts de substrat.

Les quantités ajoutées lors d'un ajout de substrat lignocellulosique prétraité représentent généralement une augmentation du taux de matière de sèche d'au plus 5 % poids, de préférence comprise entre 2 et 5 % poids, et encore plus préféré entre 2 et 3 % poids. La quantité de substrat ajouté lors d'un ajout représente par exemple 3 % poids de taux de matière sèche.

Selon une variante, à chaque ajout, le substrat lignocellulosique prétraité est ajouté en quantité égale.

Selon une variante préférée, l'ajout des enzymes dans le réacteur est effectué d'une façon séquentielle et de plus en plus espacé dans le temps. On a pu observer que l'ajout d'enzymes en mode « fed-batch » espacé permettait de maintenir une activité enzymatique sur la durée contrairement à un ajout de la totalité des enzymes dès le début de l'hydrolyse enzymatique.

Le substrat lignocellulosique prétraité et les enzymes peuvent être ajoutés en même temps ou en décalé dans le réacteur, tout en respectant l'ajout séquentiel de plus en plus espacé dans le temps de chacune des composantes. De préférence, le substrat lignocellulosique prétraité et les enzymes sont ajoutés en même temps dans le réacteur.

Selon une variante, à chaque ajout, les enzymes sont ajoutées en quantité égale.

Les quantités ajoutées lors d'un ajout sont généralement comprises entre 0,1 à 60 mg d'enzymes par gramme de cellulose, de préférence comprises entre 5 et 40 mg d'enzymes par gramme de cellulose et de manière préférée comprises entre 10 et 30 mg d'enzymes par gramme de cellulose.

L'hydrolyse enzymatique est généralement réalisée à un pH compris entre 4 et 6, de préférence compris entre 4,5 et 5,8 et encore plus préférentiellement entre 4,8 et 5,5. Elle se déroule généralement à une température entre 40 et 60 °C, et de préférence entre 45 et 55°C. Elle opère avantageusement à pression atmosphérique.

L'hydrolyse enzymatique est réalisée au moyen d'enzymes produites par un microorganisme. La solution enzymatique ajoutée contient des enzymes qui décomposent la cellulose en sucres. Des micro-organismes, comme les champignons appartenant aux genres *Trichoderma, Aspergillus, Penicillium ou Schizophyllum,* ou les bactéries anaérobies appartenant par exemple au genre *Clostridium,* produisent ces enzymes, contenant notamment les cellulases adaptées à l'hydrolyse poussée de la cellulose. De façon très préférée, les enzymes cellulolytiques de l'étape d) sont produits par le microorganisme *Trichoderma reesei.*

Conformément à l'invention, la durée de mise en contact lors de l'hydrolyse enzymatique est comprise entre 5 et 200 heures, de préférence entre 2 et 100 heures et de manière préférée entre 1 et 50 heures.

Ledit procédé selon la présente invention peut être suivi en mesurant au cours du temps la valeur de l'une des caractéristiques rhéologiques du milieu réactionnel qui sont avantageusement choisies parmi la viscosité du milieu réactionnel, le couple de torsion de l'arbre du système d'agitation et la puissance électrique consommée par le moteur. La puissance électrique consommée par le moteur est notée P_{elec}.

Durant le procédé selon l'invention, c'est à dire durant la liquéfaction, la viscosité du milieu réactionnel, le couple de torsion de l'arbre du système d'agitation et la puissance électrique consommée par le moteur sont des caractéristiques rhéologiques de suivi du substrat lignocellulosique produit qui présentent plusieurs intérêts. En effet, lesdites caractéristiques, viscosité, couple et puissance, sont reliées entre elles. La puissance électrique consommée par le moteur P_{elec} est liée à la puissance mécanique P_{méca} entrainant l'arbre d'agitation.

La puissance électrique consommée par le moteur est un paramètre classiquement mesuré et suivi sur les installations pilote ou industrielle.

Les formules suivantes définissent les relations entre les différents paramètres :
P_{méca} = f (P_{elec}), f étant une caractéristique de conception du moteur et étant donnée par le constructeur du moteur.
P_{méca} = 2πN^{∗}C dans laquelle :
   N est la vitesse d'agitation en tour par seconde,
   C est le couple en N.m,
   et P_{méca} est la puissance en watt.

En agitation on a la relation suivante :
P_{méca} = ρNₚN³D⁵
ρ est la densité du milieu réactionnel en kg.m⁻³
D est le diamètre de l'agitateur en m,
Nₚ est une caractéristique de l'agitateur dépendant de la géométrie de la cuve et du régime d'écoulement.

En régime d'écoulement laminaire, on a la relation suivante :
Nₚ=A/Re d'où P_{méca} = ρAN³D⁵/Re
avec A étant une constante du système d'agitation et Re le nombre de Reynolds et Re = ρND²/*µ̅*,
*µ̅* étant la viscosité dynamique moyenne mesurée en Pascal seconde (Pa.s) du milieu réactionnel avec *µ̅* = P_{méca}/(AN²D³) = 2πC/(AD³N)

Si la viscosité et le couple de torsion de l'arbre du système d'agitation sont des mesures facilement accessibles à petite échelle, la puissance électrique consommée par le moteur P_{elec} est la grandeur la plus facilement mesurable à échelle industrielle.

De manière très préférée, ledit procédé selon la présente invention se caractérise en ce qu'on réalise une mesure au cours du temps de la puissance électrique consommée par le moteur.

Ledit procédé selon la présente invention est avantageusement réalisé dans un réacteur, de préférence de forme cylindrique, présentant un ratio hauteur/diamètre avantageusement compris entre 1 et 3.

Grâce au mode « fed-batch » de plus en plus espacé, l'effet de la viscosité est moins important dans le milieu réactionnel. Classiquement, l'agitateur choisi doit pouvoir traiter des écoulements laminaires. Des agitateurs larges, voire raclant la paroi du réacteur avec des vitesses de rotation modérée et exerçant une action de malaxage et pétrissage sont alors nécessaires. Dans le procédé selon l'invention, des agitateurs plus simples, de type agitateurs à pâles inclinées ou encore de type hélice marine peuvent être utilisés.

On peut notamment utiliser des agitateurs ayant un diamètre plus petit dans le procédé selon l'invention. Le rapport diamètre agitateur/diamètre réacteur D/T est compris entre 0,3 et 0,75, et de préférence entre 0,4 et 0,65.

De même, la vitesse de rotation peut être plus faible que dans le système classique. La vitesse de rotation est inférieure à 100 rpm (rotations par minute), de préférence inférieure à 80 rpm.

Selon un mode de réalisation préféré, le procédé d'hydrolyse enzymatique selon l'invention peut être suivi d'une étape de fermentation alcoolique par un microorganisme alcooligène de manière à produire un effluent fermenté contenant de l'alcool.

L'hydrolyse enzymatique et la fermentation alcoolique peuvent aussi être opérées de manière simultanée. Dans ce cas on parle d'un procédé "SSF" (selon le terme anglo-saxon pour Simultaneous Saccharification and Fermentation). L'hydrolyse enzymatique et la fermentation alcoolique peuvent aussi être mises en œuvre selon d'autres agencements connus de l'homme du métier, tel que le procédé "PSSF" (Presacchararification followed by Simultaneous Saccharification and Fermentation selon le terme anglo-saxon) ou encore le procédé "HHF" (Hybrid Hydrolysis and Fermentation selon le terme anglo-saxon).

Les sucres obtenus par hydrolyse enzymatique peuvent être fermentés en alcools tel que l'éthanol, le 1,3-propanediol, l'isopropanol, le 1-butanol, l'isobutanol ou le 1,4-butanediol, seul ou en mélange. De préférence, la fermentation alcoolique produit de l'éthanol.

La fermentation alcoolique est assurée par des levures ou autres microorganismes alcooligènes. Au sens de la présente invention, le terme "fermentation alcoolique" désigne un procédé de fermentation des sucres en alcool(s) au seul moyen de microorganismes. Les microorganismes alcooligènes utilisés pendant l'étape de fermentation alcoolique des hexoses sont de préférence choisis parmi les levures et les bactéries, éventuellement génétiquement modifiées.

Lorsque le microorganisme alcooligène est une levure, *Saccharomyces cerevisiae* est celle qui est la plus performante. Il est également possible de choisir des levures telles que *Schizosaccharomyces pombe* ou *Saccharomyces uvarum* ou *diastaticus.* Des levures plus thermophiles, telles que les *Kluyveromyces fragilis* (maintenant souvent désignée par *K. marxianus*) présentent également un intérêt, notamment lorsque l'hydrolyse enzymatique et la fermentation alcoolique sont réalisées simultanément (procédé SSF).

Un organisme génétiquement modifié, comme par exemple une levure de type *Saccharomyces cerevisiae* telle que la TMB 3400 (Ohgren et al, J. of Biotech 126, 488-498, 2006) peut également être utilisé.

Lorsque le microorganisme alcooligène est une bactérie, on préférera *Zymomonas mobilis* qui présente une voie d'assimilation efficace pour la production d'éthanol, ou les bactéries anaérobies du genre *Clostridium,* comme par exemple, *Clostridium acetobutylicum* pour la production de mélanges d'alcools et solvants comme acétone-butanol-éthanol (ABE) ou isopropanol-butanol-éthanol (IBE), ou encore *Escherichia coli* pour la production d'isobutanol par exemple.

La fermentation alcoolique est réalisée préférentiellement à une température comprise entre 30°C et 40°C, et un pH entre 3 et 6,5.

Les levures, et de préférence *Saccharomyces cerevisiae* sont les microorganismes utilisés de façon très préférée. Ils présentent une meilleure robustesse, sécurité, et ne nécessitent pas de stérilité pour la conduite du procédé et des installations.

Les levures du genre *Saccharomyces* sont capables de fermenter les seuls et uniques hexoses (glucose et mannose essentiellement). Ces levures valorisent de façon optimale les hexoses en éthanol et permettent d'atteindre de bons rendements de conversion.

Lorsque l'hydrolyse enzymatique et la fermentation alcoolique sont réalisées dans une même et seule opération (procédé SSF), de préférence la température est comprise entre 30 et 45°C, et le pH compris entre 4 et 6 afin de favoriser la performance des levures.

L'exemple de fonctionnement ci-après permet d'illustrer le procédé selon l'invention.

### EXEMPLES

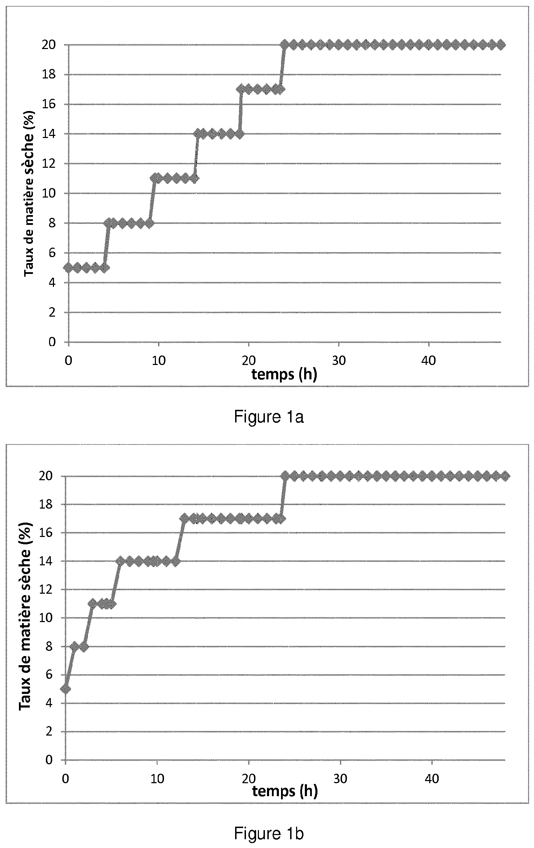

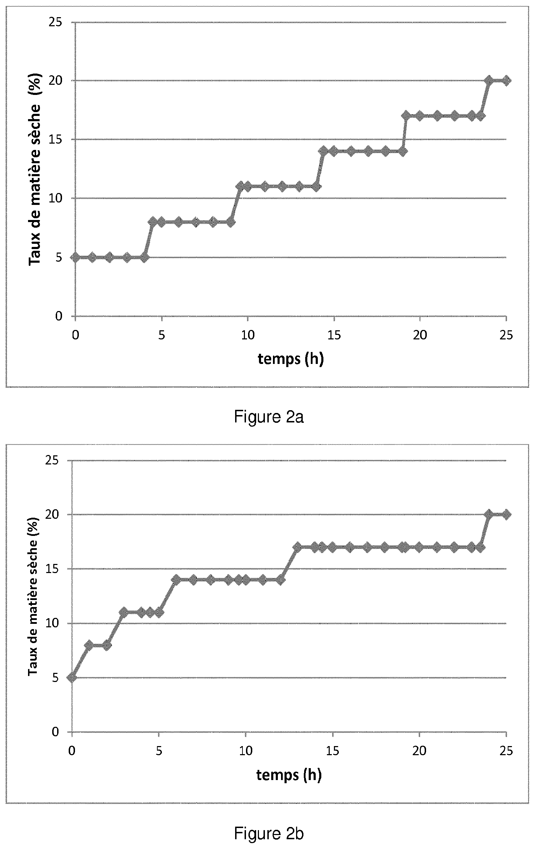

Un procédé d'hydrolyse enzymatique de paille de blé prétraitée à haute teneur en matière sèche (MS) a été effectué par alimentation séquentielle (fed-batch) de façon de plus en plus espacée. A la différence des stratégies conventionnelles de fed-batch, où les ajouts sont réguliers dans le temps, cette nouvelle stratégie consiste à augmenter séquentiellement la teneur en matière sèche avec des ajouts qui sont de plus en plus espacés dans le temps. Les Figures 1a et 2a montrent des ajouts selon la technique fed-batch conventionnel, alors que les figures 1b et 2b montrent des ajouts selon la technique fed-batch de plus en plus espacés dans le temps. Les figures 2 sont des agrandissements des figures 1 au niveau de l'échelle du temps.

Toute la teneur en eau (1,3 kg) et une première addition de substrat paille de blé prétraité (250 g), suffisante pour arriver à une concentration de 5% poids de matière sèche, sont chargées dans le réacteur au début du test. Ensuite, cinq ajouts égaux de 170 g de substrats ligno-cellulosiques se produisent après 1, 3, 6, 13 et 24 heures pour arriver à 20% poids de matière sèche (figures 1b et 2b). L'addition des enzymes a été effectuée de la même manière. Avec cette stratégie, une liquéfaction graduelle des particules des substrats est possible, sans surmonter la valeur critique de viscosité qui ne permettait pas un mélange adéquat avec des agitateurs à pâles inclinées ou encore de type hélice marine.

Le procédé selon l'invention permet d'atteindre un rendement de glucose de 80 à 85%, avec une faible consommation énergétique entre 35-40 kJ dans 48 h d'hydrolyse enzymatique. En particulier, la consommation énergétique obtenue par le procédé selon l'invention en 48 h était la même que celle obtenue en seulement 5 h par d'autres stratégies de fed-batch. En effet, on observe que le glucose a une augmentation exponentielle dans les premières 24 heures du test, où il y a une activité intense de conversion de la cellulose par la cellulase. Après, la croissance est plus lente: dans les premières heures, une augmentation de 50 à 90 g de glucose a été enregistrée pour chaque kJ consommé dans le mélange; après 24 heures, il était de 10-15 g / kJ et après 48h, il a diminué de moins de 10 g / kJ.

De même, la vitesse de rotation du système de mélange est lente (environ 80 tr / min). Les enzymes sont des protéines avec une structure moléculaire, stabilisée par des forces faibles. Cette stabilisation faible implique que les protéines sont affectées par des paramètres différents. Le stress mécanique est un facteur qui peut réduire l'activité enzymatique.

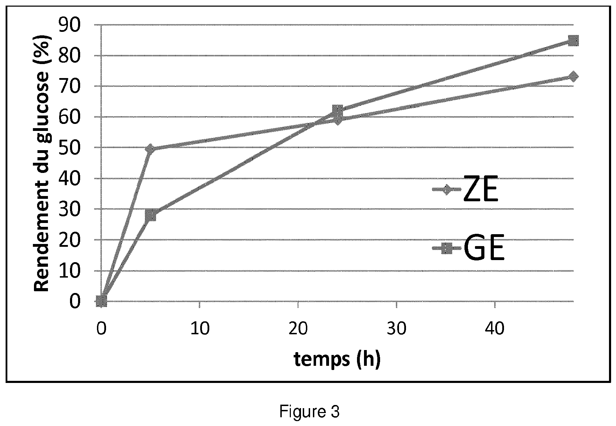

La stratégie des ajouts séquentiels de plus en plus espacés dans le temps peut être adoptée aussi pour les enzymes. La Figure 3 montre la différence entre une addition de toute la quantité des enzymes au début de test (ZE, Figure 3) et des ajouts séquentiels, en concomitance avec les ajouts séquentiels des substrats (GE, Figure 3).

La figure 3 montre que la croissance du glucose est très rapide si la quantité d'enzymes (52,8 g) est en totalité alimentée au début de l'hydrolyse enzymatique. Cependant, ceci implique une production de glucose trop rapide dans le milieu réactionnel ce qui cause l'inhibition des enzymes et par conséquent de la production de glucose. Au contraire, l'addition progressive d'enzymes (8,8 g pour chaque ajout, de plus en plus espacé dans le temps) permet de remplacer les enzymes inhibées et d'obtenir une production de glucose plus élevée à la fin du procédé (Figure 3).

## Revendications

1. Procédé d'hydrolyse enzymatique en alimentation séquentielle dans lequel on met en contact, sous agitation, au moins un substrat lignocellulosique prétraité avec de l'eau et avec des enzymes dans un réacteur, ledit procédé étant **caractérisé en ce que** l'ajout séquentiel dans le réacteur du substrat lignocellulosique prétraité est effectué de façon de plus en plus espacée dans le temps, de manière à obtenir un taux de matière sèche final prédéterminé compris entre 18 et 30% poids,
dans lequel :
le taux de matière sèche en début du procédé est inférieur à 10 % poids,
le réacteur comprend un agitateur, la vitesse de rotation dudit agitateur étant inférieure à 100 rpm et le rapport diamètre de l'agitateur/diamètre du réacteur D/T étant compris entre 0,3 et 0,75.

2. Procédé selon la revendication 1, dans lequel le taux de matière sèche final est compris entre 18 et 24% poids.

3. Procédé selon l'une des revendications précédentes, **caractérisé en ce qu'**on procède à au moins 4 ajouts espacés de substrat lignocellulosique prétraité, et de préférence à au moins 5 ajouts.

4. Procédé selon l'une des revendications précédentes, **caractérisé en ce que** la quantité de substrat lignocellulosique ajoutée lors d'un ajout représente entre 2 et 3% poids de matière sèche.

5. Procédé selon l'une des revendications précédentes, dans lequel l'ajout des enzymes dans le réacteur est effectué d'une façon séquentielle et de plus en plus espacé dans le temps.

6. Procédé selon l'une des revendications précédentes, dans lequel le substrat lignocellulosique prétraité et les enzymes sont ajoutés en même temps dans le réacteur.

7. Procédé selon l'une des revendications précédentes, dans lequel, à chaque ajout, le substrat lignocellulosique prétraité est ajouté en quantité égale.

8. Procédé selon l'une des revendications précédentes, dans lequel à chaque ajout, les enzymes sont ajoutées en quantité égale.

9. Procédé selon l'une des revendications précédentes, dans lequel l'agitateur est un agitateur à pâles inclinées ou de type hélice marine.

10. Procédé selon l'une des revendications précédentes, **caractérisé en ce que** le réacteur comprend un agitateur entrainé par un moteur et **en ce qu'**on réalise une mesure au cours du temps de la puissance électrique consommée par le moteur.

11. Procédé selon l'une des revendications précédentes, **caractérisé en ce que caractérisé en ce que** le réacteur comprend un agitateur entrainé par un moteur et **en ce que** la vitesse de rotation de l'agitateur est inférieure à 80 rpm.

12. Procédé selon l'une des revendications précédentes, dans lequel les enzymes sont mises en contact à une concentration comprise entre 0,1 et 60 mg d'enzymes par gramme de cellulose.

13. Procédé selon l'une des revendications précédentes, dans lequel le procédé opère à une température comprise entre 40 et 60°C, à un pH compris entre 4 et 6, et à pression atmosphérique.

14. Procédé selon l'une des revendications précédentes, dans lequel différents substrats lignocellulosiques prétraités sont utilisés, seuls ou en mélange.

15. Procédé selon l'une des revendications précédentes, dans lequel ledit procédé est suivi d'une étape de fermentation en présence d'un microorganisme alcooligène.

16. Procédé selon l'une des revendications précédentes, dans lequel ledit procédé est réalisé en présence d'un microorganisme alcooligène selon un procédé de saccharification et de fermentation simultanées.

## Patentansprüche

1. Enzymatisches Hydrolyseverfahren mit sequentieller Zufuhr, wobei unter Rühren mindestens ein vorbehandeltes Lignozellulosesubstrat mit Wasser und mit Enzymen in einem Reaktor in Kontakt gebracht wird, wobei das Verfahren **dadurch gekennzeichnet ist, dass** die sequentielle Zugabe des vorbehandelten Lignozellulosesubstrats in den Reaktor in immer größeren Zeitabständen erfolgt, damit ein vorher festgelegter Endtrockensubstanzgehalt zwischen 18 und 30 Gewichts-% erhalten wird,
wobei:
der Trockensubstanzgehalt am Anfang des Verfahrens unter 10 Gewichts-% liegt,
wobei der Reaktor einen Rührer umfasst, die Drehzahl des Rührers unter 100 U/min liegt und das Verhältnis von Durchmesser des Rührers/Durchmesser des Reaktors D/T zwischen 0,3 und 0,75 liegt.

2. Verfahren nach Anspruch 1, wobei der Endtrockensubstanzgehalt zwischen 18 und 24 Gewichts-% liegt.

3. Verfahren nach einem der vorhergehenden Ansprüche, **dadurch gekennzeichnet, dass** mindestens 4 Zugaben, und vorzugsweise mindestens 5 Zugaben, von vorbehandeltem Lignozellulosesubstrat im Abstand erfolgen.

4. Verfahren nach einem der vorhergehenden Ansprüche, **dadurch gekennzeichnet, dass** die bei einer Zugabe zugegebene Menge an Lignozellulosesubstrat zwischen 2 und 3 Gewichts-% Trockenmasse ausmacht.

5. Verfahren nach einem der vorhergehenden Ansprüche, wobei die Zugabe der Enzyme in den Reaktor sequentiell und in immer größeren Zeitabständen erfolgt.

6. Verfahren nach einem der vorhergehenden Ansprüche, wobei das vorbehandelte Lignozellulosesubstrat und die Enzyme gleichzeitig in den Reaktor zugegeben werden.

7. Verfahren nach einem der vorhergehenden Ansprüche, wobei bei jeder Zugabe das vorbehandelte Lignozellulosesubstrat in derselben Menge zugegeben wird.

8. Verfahren nach einem der vorhergehenden Ansprüche, wobei bei jeder Zugabe die Enzyme in derselben Menge zugegeben werden.

9. Verfahren nach einem der vorhergehenden Ansprüche, wobei der Rührer ein Schrägblattrührer oder ein Propellerrührer ist.

10. Verfahren nach einem der vorhergehenden Ansprüche, **dadurch gekennzeichnet, dass** der Reaktor einen von einem Motor angetriebenen Rührer umfasst, und dadurch, dass eine Messung der von dem Motor verbrauchten elektrischen Leistung über den Zeitverlauf vorgenommen wird.

11. Verfahren nach einem der vorhergehenden Ansprüche, **dadurch gekennzeichnet, dass** der Reaktor einen von einem Motor angetriebenen Rührer umfasst, und dadurch, dass die Drehzahl des Rührers unter 80 U/min liegt.

12. Verfahren nach einem der vorhergehenden Ansprüche, wobei die Enzyme bei einer Konzentration zwischen 0,1 und 60 mg Enzyme pro Gramm Cellulose in Kontakt gebracht werden.

13. Verfahren nach einem der vorhergehenden Ansprüche, wobei das Verfahren bei einer Temperatur zwischen 40 und 60°C, bei einem pH-Wert zwischen 4 und 6 und bei Luftdruck durchgeführt wird.

14. Verfahren nach einem der vorhergehenden Ansprüche, wobei verschiedene vorbehandelte Lignozellulosesubstrate, allein oder gemischt, verwendet werden.

15. Verfahren nach einem der vorhergehenden Ansprüche, wobei auf das Verfahren ein Schritt zur Fermentierung in Gegenwart eines alkoholerzeugenden Mikroorganismus folgt.

16. Verfahren nach einem der vorhergehenden Ansprüche, wobei das Verfahren in Gegenwart eines alkoholerzeugenden Mikroorganismus gemäß einem Verfahren zur gleichzeitigen Verzuckerung und Fermentierung durchgeführt wird.

## Claims

1. Process for enzymatic hydrolysis by sequential feeding in which, under stirring, at least one pretreated lignocellulosic substrate is brought into contact with water and with enzymes in a reactor, said process being **characterized in that** the sequential addition to the reactor of the pretreated lignocellulosic substrate is carried out increasingly spaced out over time, so as to obtain a predetermined final solids content of between 18% and 30% by weight,
in which:
the solids content at the start of the process is less than 10% by weight,
the reactor comprises an impeller, the speed of rotation of said impeller being less than 100 rpm and the diameter of the impeller/diameter of the reactor ratio D/T being between 0.3 and 0.75.

2. Process according to Claim 1, in which the final solids content is between 18% and 24% by weight.

3. Process according to either of the preceding claims, **characterized in that** at least 4 spaced-out additions of pretreated lignocellulose substrate, preferably at least 5 additions, are carried out.

4. Process according to one of the preceding claims, **characterized in that** the amount of lignocellulosic substrate added during an addition represents between 2% and 3% by weight of solids.

5. Process according to one of the preceding claims, in which the addition of the enzymes to the reactor is carried out sequentially and increasingly spaced-out over time.

6. Process according to one of the preceding claims, in which the pretreated lignocellulosic substrate and the enzymes are added at the same time to the reactor.

7. Process according to one of the preceding claims, in which, at each addition, the pretreated lignocellulosic substrate is added in the same amount.

8. Process according to one of the preceding claims, in which, at each addition, the enzymes are added in the same amount.

9. Process according to one of the preceding claims, in which the impeller is an inclined blade impeller or an impeller of marine propeller type.

10. Process according to one of the preceding claims, **characterized in that** the reactor comprises an impeller driven by a motor and **in that** a measurement is carried out over time of the electric power consumed by the motor.

11. Process according to one of the preceding claims, **characterized in that** the reactor comprises an impeller driven by a motor and **in that** the speed of rotation of the impeller is less than 80 rpm.

12. Process according to one of the preceding claims, in which the enzymes are brought into contact at a concentration of between 0.1 and 60 mg of enzymes per gram of cellulose.

13. Process according to one of the preceding claims, in which the process takes place at a temperature of between 40°C and 60°C, at a pH of between 4 and 6, and at atmospheric pressure.

14. Process according to one of the preceding claims, in which various pretreated lignocellulosic substrates are used, alone or as a mixture.

15. Process according to one of the preceding claims, in which said process is followed by a step of fermentation in the presence of an alcohol-producing microorganism.

16. Process according to one of the preceding claims, in which said process is carried out in the presence of an alcohol-producing microorganism according to a process of simultaneous saccharification and fermentation.
